# EUROPEAN PATENT SPECIFICATION

(11) **EP 1 293 780 B1**
(45) Date of publication and mention of the grant of the patent: **28.06.2006**
(21) Application number: 02020330.3
(22) Date of filing: 11.09.2002
(51) Int. Cl.: G01N 33/543, G01N 33/558, G01N 33/58

(54) **Specific binding analysis method**
Spezifisches Bindungsanalyseverfahren
Méthode d'analyse de liaisons spécifiques

(30) Priority: 14.09.2001 JP 2001280359
(43) Date of publication of application: 19.03.2003
(73) Proprietor: MATSUSHITA ELECTRIC INDUSTRIAL CO., LTD., Kadoma-shi, Osaka 571-8501 (JP)
(72) Inventor: Kawamura, Tatsurou, Kadoma-shi, Osaka 571-8501 (JP); Kamei, Akihito, Kadoma-shi, Osaka 571-8501 (JP); Kitawaki, Fumihisa, Kadoma-shi, Osaka 571-8501 (JP); Kenjyou, Noriko, Kadoma-shi, Osaka 571-8501 (JP)
(74) Representative: TBK-Patent

(56) References cited:
- JENKINS PHILIP ET AL: "Ultrasound pre-treatment can extend immunoagglutination test sensitivity while avoiding the prozone phenomenon" JOURNAL OF CLINICAL LIGAND ASSAY, vol. 22, no. 4, January 1999 (1999-01), pages 410-412, XP009019287 ISSN: 1081-1672
- SALAMA A ET AL: "ELIMINATION OF THE PROZONE EFFECT IN THE ANTI GLOBULIN REACTION BY A SIMPLE MODIFICATION" VOX SANGUINIS, vol. 42, no. 3, 1982, pages 157-159, XP009019286 ISSN: 0042-9007

## Description

### BACKGROUND OF THE INVENTION

The present invention relates to a specific binding analysis method for performing a qualitative or quantitative analysis of an analyte in a sample.

With the recent expansion of medical care in households and communities as well as increase of clinical examinations requiring high urgency, there is an increasing demand for the development of a specific binding analysis method which can be performed even by persons other than the experts of the clinical examination, in a rapid, simple and accurate manner.

Many methods are known as the conventional specific binding analyses, which include immunoassay utilizing an antigen-antibody reaction, receptor assay employing a receptor and nucleic acid probe assay employing the hybridization of complementary nucleic acid sequences. Because of their high specificity, these methods are being widely used in the clinical examinations and in many other fields.

In chromatography, which is a type of immunoassay, a liquid sample is brought into contact with a matrix comprising, for example, a porous carrier or a fine particle-packed carrier in each of which a specific binding substance is insolubilized or immobilized. Then, the presence or absence of an analyte in the sample is analyzed by utilizing a phenomenon in which the liquid sample flows out along the matrix by permeating force caused by capillarity (see, e.g., Japanese Patent Nos. 2504923 and 2667793, Japanese Examined Patent Publication No. Hei 7-78503, Japanese Unexamined Patent Publication Nos. Hei 10-73592 and Hei 8-240591).

More specifically, a specific binding substance, which is labeled with a labeling material freely detectable by naked eyes or with an optical method, is specifically bound to an analyte. The specific binding substance specifically bound to the analyte is then bound to a binding material immobilized on the matrix. Finally, the presence or absence of the analyte in the sample is analyzed, according to the labeled amount of the specific binding substance immobilized on the matrix.

The carrier comprising the matrix used for such chromatography has a large surface area where a great amount of a specific binding substance can be immobilized, so that the collision between reacting molecules, which may cause a specific binding reaction, occurs with a higher frequency as compared with the reaction in a liquid phase. Accordingly, the above-described chromatography is advantageous from the viewpoint of the measurement sensitivity and the measurement time.

However, the conventional chromatography has a problem called a prozone phenomenon. The prozone phenomenon is a phenomenon in which the concentration of an analyte cannot be unambiguously determined from a signal intensity attributed to a specific binding reaction.

Specifically, when an excessive amount of an analyte is present in a sample, there are present on a matrix an analyte specifically bound to a labeled specific binding substance (a labeling material-specific binding substance-analyte complex), and an analyte not bound to the labeled specific binding substance (a simple substance).

The analyte bound to the labeled specific binding substance and the analyte as the simple substance compete to specifically bind to a binding material immobilized on the matrix. Thus, there is a case where the analyte as the simple substance is undesirably bound to the binding material, and the analyte bound to the labeled specific binding substance is flowed out due to permeating force caused by capillarity. This results in a reduced amount of the labeled specific binding substance bound to the binding material, so that the signal intensity attributed to the specific binding reaction of the specific binding substance does not correspond to the amount of the analyte contained in the sample.

Accordingly, when an excessive amount of an analyte is present in a sample, the amount of the analyte determined from the labeled amount of the specific binding substance is measured to be abnormally small, thereby in some cases preventing an accurate determination of the presence or absence of the analyte in the sample.

In order to accurately determine the amount of an analyte without being influenced by the prozone phenomenon, there has been proposed a method of confirming the presence or absence of an analyte having a high concentration by diluting samples to various concentrations and measuring the signals of the respective samples plural times. However, this method requires the use of a plurality of reaction vessels and therefore renders the measurement steps complicated, resulting in a problem of increasing the size and complexity of the analysis device.

Therefore, it is an object of the present invention to provide a specific binding analysis method which enables accurate qualitative and quantitative analyses of an analyte in a sample without being influenced by the prozone phenomenon, even with the use of a small, simple measurement device, and a specific binding analysis device used therefor. According to the present invention, measurement accuracy is high, particularly in the low concentration range, because a labeling material-first specific binding substance-analyte complex is immobilized in a first detection zone where a second specific binding substance is immobilized, and a quantitative analysis is performed based on the intensity of a signal in the first detection zone.

### BRIEF SUMMARY OF THE INVENTION

The present invention relates to a specific binding analysis method comprising the steps of:
(A) bringing into contact a sample containing an analyte with a first specific binding substance which specifically binds to the analyte and is labeled with a detectable labeling material, to allow the analyte to bind to the first specific binding substance, thereby obtaining a labeling material-first specific binding substance-analyte complex;
(B) bringing into contact the sample subjected to step (A) with a first detection zone where a second specific binding substance is immobilized, to allow an excess of the analyte, which has not bound to the first specific binding substance, and the complex to bind to the second specific binding substance, thereby immobilizing the excess of the analyte and the complex in the first detection zone;
(C) bringing into contact the sample subjected to step (B) a second detection zone where a substance identical to the analyte or an analog of the analyte is immobilized, to allow the first specific binding substance in the complex to bind to the substance or the analog, thereby immobilizing the complex in the second detection zone;
(D) respectively measuring an intensity of signal 1 and an intensity of signal 2, which are attributed to the labeling material and are obtained in the first detection zone and the second detection zone; and
(E) determining a concentration of the analyte in the sample based on the intensity of the signal 1 and the intensity of the signal 2, thereby performing a qualitative or quantitative analysis of the analyte in the sample.

It is preferable that in the step (E), when the intensity of the signal 1 obtained in step (D) corresponds to different concentrations of the analyte based on quantitative information previously obtained from a sample having a known concentration of the analyte, a concentration of the analyte is determined after judging which concentration is true based on the intensity of the signal 2.

It is preferable that the above-described specific binding analysis method further comprises judging whether or not a prozone phenomenon is present based on the intensity of the signal 2 obtained in the step (D).

It is preferable that in the step (B), the sample having been subjected to the step (A) is brought into contact with a first detection zone where a second specific binding substance is immobilized, to allow an excess of the analyte, which has not bound to the first specific binding substance, and the complex to bind to the second specific binding substance, thereby immobilizing the excess of the analyte and the complex in the first detection zone, and that
in the step (C), the sample having been subjected to the step (B) is brought into contact with a second detection zone where a substance identical to the analyte or an analog of the analyte is immobilized, to allow the first specific binding substance in the complex to bind to the substance or the analog, thereby immobilizing the complex in the second detection zone,
the step (C) being performed subsequently to the step (B).

It is also preferable that in the step (C), the sample having been subjected to the step (A) is brought into contact with a second detection zone where a substance identical to the analyte or an analog of the analyte is immobilized, to allow the first specific binding substance in the complex to bind to the substance or the analog, thereby immobilizing the complex in the second detection zone, and that
in the step (B), the sample having been subjected to the step (C) is brought into contact with a first detection zone where a second specific binding substance is immobilized, to allow an excess of the analyte, which has not bound to the first specific binding substance, and the complex to bind to the second specific binding substance, thereby immobilizing the excess of the analyte and the complex in the first detection zone,
the step (B) being performed subsequently to the step (C).

It is also preferable that, after previously performing the step (A) and providing the first detection zone and the second detection zone on a sheet-shaped chromatography matrix, the sample having been subjected to the step (A) is dropped upstream from the first detection zone and the second detection zone, to allow the sample to migrate, by permeating force caused by capillarity, to each of the first detection zone and the second detection zone on the matrix, followed by performing the steps (B) to (E).

Further, it is preferable that, after providing a retention zone where a first specific binding substance labeled with a detectable labeling material is retained, the first detection zone and the second detection zone on a sheet-shaped chromatography matrix, the sample is dropped to the retention zone or upstream therefrom, to allow the sample to migrate, by permeating force caused by capillarity, to each of the retention zone, the first detection zone and the second detection zone on the matrix, followed by performing the steps (A) to (E).

It is preferable that the signal attributed to the labeling material is a signal exhibiting coloration, fluorescence or luminescence.

It is preferable that at least one of the first specific binding substance and the second specific binding substance is an antibody.

It is preferable that the labeling material is a particle containing a metal sol, a dye sol or a fluorescent substance, or a colored latex particle.

It is also preferable that in the step (D), the intensity of the signal 1 and the intensity of the signal 2 are continuously measured in an order in which the intensity of the signal 1 is measured before measuring the intensity of the signal 2, along the migrating direction of the analyte.

It is preferable that in the step (D), a graph showing the relation between a position of the analyte in the migrating direction thereof and each of the intensity of the signal 1 and the intensity of the signal 2 each measured continuously, is produced, and that
in the step (E), a qualitative or quantitative analysis is performed based on the graph.

The graph as used herein refers to either linear line or curved line.

It is preferable that in the step (E), a quantitative analysis is performed based on a quantitative information previously obtained from a sample having a known concentration of an analyte, as well as on the intensity of the signal 1 and the intensity of the signal 2 each obtained in the step (D).

Further, the present invention provides a specific binding analysis device comprising:
a sheet-shaped chromatography matrix;
a first detection zone where a specific binding substance which specifically binds to an analyte is immobilized;
a second detection zone where a substance identical to the analyte or an analog of the analyte is immobilized,
each of the first detection zone and the second detection zone being provided on the matrix,
wherein said device being adapted such that a sample containing an analyte and a specific binding substance migrate, by permeating force caused by capillarity, to each of the first detection zone and the second detection zone to cause a specific binding reaction, and a signal attributed to the specific binding reaction is detected to perform a qualitative or quantitative analysis of the analyte in the sample.

It is preferable that the above-described specific binding analysis device further comprises a retention zone where a specific binding substance labeled with a detectable labeling material, the retention zone being provided upstream from the first detection zone on the matrix,
wherein said device being adapted such that the sample migrates, by permeating force caused by capillarity, from the retention zone to each of the first detection zone and the second detection zone.

It is also preferable that the above-described specific binding analysis device further comprises a sample application zone provided upstream from the retention zone on the matrix,
wherein said device being adapted such that the sample migrates, by permeating force caused by capillarity, from the sample application zone to each of the retention zone, the first detection zone and the second detection zone.

It is also preferable that the above-described specific binding analysis device further comprises a sample application zone provided upstream from the first detection zone on the matrix,
wherein said device being adapted such that the sample migrates, by permeating force caused by capillarity, from the sample application zone to each of the first detection zone and the second detection zone.

While the novel features of the invention are set forth particularly in the appended claims, the invention, both as to organization and content, will be better understood and appreciated, along with other objects and features thereof, from the following detailed description taken in conjunction with the drawings.

### BRIEF DESCRIPTION OF THE SEVERAL VIEWS OF THE DRAWING

FIG. 1 is a schematic oblique view of one embodiment of a test reagent strip used in the specific binding analysis method of the present invention.
FIG. 2 is a schematic diagram showing the structure of one embodiment of an analysis device used in the specific binding analysis method of the present invention.
FIG. 3 is a schematic diagram showing the distribution of the signal intensity detected with the analysis device shown in FIG. 2 in a first detection zone 5 of a strip 1.
FIG. 4 is a schematic diagram showing the distribution of the signal intensity detected with the analysis device shown in FIG. 2 in a second detection zone 6 of the strip 1.
FIG. 5 is a graph showing the relation between signal intensity Hs and the hCG concentration in the case of applying light having a wavelength of 650 nm to the first detection zone 5.
FIG. 6 is a graph showing the relation between signal intensity Hs and the hCG concentration in the case of applying light having a wavelength of 650 nm to the second detection zone 6.

### DETAILED DESCRIPTION OF THE INVENTION

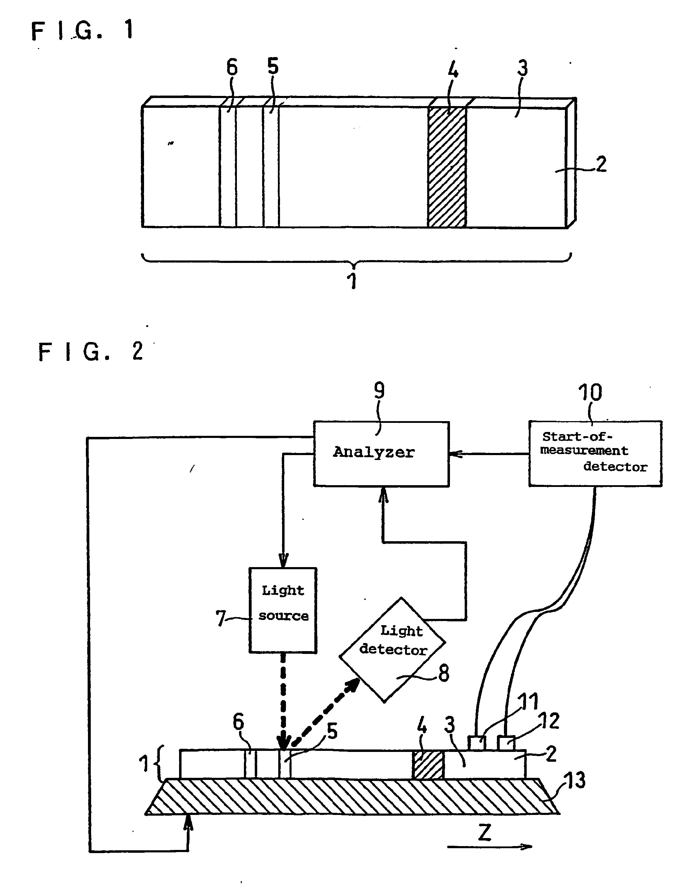

Firstly, the specific binding analysis method in accordance with the present invention will be described with reference to drawings. FIG. 1 is a schematic oblique view of a test strip which can be used as a specific binding analysis device in the specific binding analysis method of the present invention.

A test strip 1 is a sheet-shaped test strip, for example, a chromatography test strip having a matrix 2, which is a region where a sample containing an analyte can permeate and develop, formed on the surface thereof. The strip 1 comprises, for example, a porous carrier or a fine particle-packed carrier in each of which the sample can permeate and develop by capillarity.

Disposed on the matrix 2 are a sample application zone 3, a retention zone 4, a first detection zone 5 and a second detection zone 6. The sample application zone 3 is a region where the sample is applied on the matrix 2. The retention zone 4 is placed between the sample application zone 3 and a region including the first detection zone 5 and the second detection zone 6. The retention zone 4 is a region where the applied sample flows in and a first specific binding substance labeled with a labeling material is provided. The first detection zone 5 is a region where the sample that has passed through the retention zone 4 flows in and a second specific binding substance as a binding material is immobilized. The second detection zone 6 is a region where the sample that has passed through the retention zone 4 and subsequently through the first detection zone 5 flows in and an analyte or analog thereof is immobilized.

Herein, the sample used in the specific binding analysis method of the present invention is a liquid sample suspected of containing an analyte. Examples include urine, blood serum, blood plasma, whole blood, saliva, lacrimal fluid, spinal fluid and secretion from papillae. The sample may also be one prepared by suspending or dissolving a solid substance, a gel substance or sol substance of mucus, human body tissue, cell or the like, in a liquid such as a buffer solution, extract solution or dissolved solution.

The analyte used in the present invention may be any substance having a specific binding partner capable of specifically binding thereto. Examples include various proteins, polypeptides, glycoproteins, polysaccharides, complex glycolipids, nucleic acids, effector molecules, receptor molecules, enzymes and inhibitors, each of which functions as an antibody or antigen. More specific examples include: tumor markers such as α-fetoprotein, carcinoembryonic antigen (CEA), CA 125 and CA 19-9; various proteins, glycoproteins or complex glycolipids such as β2-microglobulin (β2m) and ferritin; various hormones such as estradiol (E2), estriol (E3), human chorionic gonadotropin (hCG), luteinizing hormone (LH) and human placental lactogen (hPL); various virus-associated antigens or virus-associated antibodies such as HBs antigen, HBs antibody, HBc antigen, HBc antibody, HCV antibody and HIV antibody; various allergens and IgE antibodies thereto; narcotic drugs, medical drugs and metabolites thereof; and virus-associated and tumor-associated nucleic acids having a polynucleotide sequence.

The specific binding substance used in the present invention may be any substance capable of specifically binding to the above-described analytes. Examples include antibodies, antigens, glycoproteins, polysaccharides, complex glycolipids, nucleic acids, effector molecules, receptor molecules, enzymes and inhibitors.

In the present invention, it is preferable that the first specific binding substance labeled with a labeling material and the second specific binding substance are employed, and that the labeled first specific binding substance and the second specific binding substance are able to bind to each other, via the analyte. Additionally, in terms of high specificity, at least one of the first specific binding substance and the second specific binding substance is preferably an antibody, and more preferably a monoclonal antibody.

Herein, the first specific binding substance and the second specific binding substance may not necessarily be same substances. In addition, when the analyte does not have a plurality of same epitopes, it is preferable that the first specific binding substance and the second specific binding substance have different specificities against respective different epitopes. Of course, when the analyte has a plurality of same epitopes, same substances may be used as the first specific binding substance and second specific binding substance.

The substance to be immobilized in the second detection zone 6 may be the analyte itself or analog thereof exhibiting the same behavior as that of the analyte. In other words, any substance capable of binding to the first specific binding substance labeled with a labeling material, may be employed. For example, a substance having the same epitope as that of the analyte to be bound to the first specific binding substance, may also be employed.

The labeling material used in the present invention may be any substance whose presence can be freely detected. Examples include not only a direct label such as a labeling material visible by naked eyes in the natural state, a labeling material visible with the use of an optical filter or a labeling material visible when stimulated with an ultraviolet ray or the like to promote its fluorescence, but also an indirect label such as a labeling material whose visible signal is detectable by adding therewith a developing reagent such as a substrate.

Examples of the direct label include fine colored particles such as dye sols, metal sols or colored latex particles, and particles containing a fluorescent substance. On the other hand, examples of the indirect label include enzymes such as alkaline phosphatase and horseradish peroxidase. The direct label may be preferably used, since it generates a signal detectable without addition of a different reagent and thereby instantaneously provides an analytical result, as well as being durable and stable.

In one embodiment of the present invention described below, urine was used as a sample and an hCG was used as an analyte contained in the sample. Further, an anti-hCG monoclonal antibody capable of participating in a sandwich reaction with the hCG was used both as a first specific binding substance and as a second specific binding substance, and colloidal gold was used as a labeling material.

Herein, the use of colored particles of colloidal gold or the like allows a labeled portion to be concentrated in a small zone or volume, because the colored particles are minute. This enables an accurate qualitative and/or quantitative analysis of the hCG to be performed in the first detection zone 5 and the second detection zone 6, using a signal attributed to a reaction in which colloidal gold serving as the labeling material for the first specific binding substance participates.

The sample to be subjected to a qualitative or quantitative analysis is firstly applied to the sample application zone 3. The applied sample migrates from the sample application zone 3 to the retention zone 4 in the matrix 2, while permeating and developing by capillarity. That is, the sample containing an analyte is brought into contact with the retention zone retaining the first specific binding substance labeled with a detectable labeling material, to allow the analyte to bind to the first specific binding substance, thereby obtaining a labeling material-first specific binding substance-analyte complex (step (A)).

As described above, any material capable of forming a site where the analyte and the specific binding substance can develop may be employed as the material constituting the matrix 2. Examples include a porous carrier, a gel carrier and a fine particle-packed carrier. In this embodiment, nitrocellulose is employed.

Nitrocellulose is superior to other matrix materials such as paper, because it is inherently capable of binding to protein without being previously sensitized. When directly applied to nitrocellulose, a specific binding substance such as an antibody can be reliably immobilized, without requiring any chemical treatment which might suppress the specific binding capability of the specific binding substance. On the other hand, when paper is used as the matrix material, for example, the immobilization of the antibody necessitates a chemical binding to be performed using CNBr, carbonyldiimidazole, tressil chloride or the like.

Moreover, nitrocellulose is commercially available in various pore sizes, so that the matrix material can be readily selected according to requirements such as the flow rate of sample. In the case of using a nitrocellulose sheet, it is preferable to improve the strength and handleability by attaching (backing) on the back of the nitrocellulose sheet, a sheet-like reinforcing material made of plastic or the like. Such a reinforced nitrocellulose sheet can be readily produced by forming a thin film of nitrocellulose on a reinforcing material.

After the antibody is immobilized in the detection zone, it is preferable to block the matrix to reduce nonspecific absorption to the matrix. The blocking may be performed by application of, for example, protein (e.g., bovine serum albumin and milk protein), polyvinylalcohol or ethanolamine, or a combination thereof.

Provided in the retention zone 4 is the anti-hCG monoclonal antibody as the first specific binding substance capable of immunologically binding to the hCG as the analyte. Herein, the anti-hCG monoclonal antibody is labeled with colloidal gold serving as the labeling material.

The hCG as the analyte contained in a urine sample flows into the retention zone 4, and it specifically binds to the first specific binding substance therein to give a labeling material-first specific binding substance-analyte complex. In other words, the hCG as the analyte is provided with colloidal gold via the anti-hCG monoclonal antibody.

Herein, it is preferable to provide in the retention zone 4, the first specific binding substance in the dry state. For example, after blocking the matrix, a reagent containing the first specific binding substance is applied to the matrix, followed by a freeze-drying treatment, thereby retaining in the retention zone 4, the first specific binding substance in the dry state. As a result, when the retention zone 4 is in the dry state, the first specific binding substance is retained in the retention zone 4. When the matrix 2 is wetted with the liquid sample permeating and developing therein, the first specific binding substance can freely migrate on the matrix 2.

Accordingly, the sample containing the complex, in which the analyte is bound to the first specific binding substance, flows into the first detection zone 5 and the second detection zone 6 provided on the matrix 2, while permeating and developing in the matrix 2.

In the first detection zone 5, the anti-hCG monoclonal antibody as the second specific binding substance capable of binding to the hCG as the analyte, is substantially immobilized. For example, when the matrix comprises nitrocellulose, the antibody can be immobilized by applying to the matrix a reagent containing the above antibody to allow the antibody to be absorbed in the matrix, both physically and chemically. This anti-hCG monoclonal antibody as the second specific binding substance does not migrate even when it is in the wet state.

In the second detection zone 6, the analyte (hCG) or analog thereof capable of binding to the anti-hCG monoclonal antibody as the first specific binding substance, is substantially immobilized. This analyte and analog thereof also do not migrate when they are in the wet state.

It should be noted that the sample may be applied to the sample application zone after being reacted with the first specific binding substance outside the test reagent strip. The first specific binding substance to be reacted with the sample outside the strip may be the one not bound to the labeling material. Further, it may also be the one bound to a labeling material which generates a signal different from that of the labeled first specific binding substance in the matrix. When the labeled first specific binding substance is previously reacted with the sample outside the strip, it is not necessary to provide on the matrix 2, the retention zone 4 where the first specific binding substance is provided.

The analyte in the sample that has reached the first detection zone 5 specifically binds to the second specific binding substance. As a result, the analyte is immobilized in the first detection zone 5, via the second specific binding substance. More specifically, the colloidal gold labeled anti-hCG monoclonal antibody as the first specific binding substance binds, via the hCG as the analyte, to the anti-hCG monoclonal antibody as the second specific binding substance immobilized in the first detection zone 5.

That is, the sample having been subjected to the step (A) is brought into contact with a first detection zone where a second specific binding substance is immobilized, to allow an excess of the analyte, which has not been bound to the first specific binding substance, to bind to the second specific binding substance, thereby immobilizing the excess of the analyte in the first detection zone (step (B)).

Herein, when an excessive amount of the hCG is present in the sample, the hCG not specifically bound to the colloidal gold labeled anti-hCG monoclonal antibody and the hCG specifically bound to the colloidal gold labeled anti-hCG monoclonal antibody compete to specifically bind to the second specific binding substance in the first detection zone 5.

As a result, the hCG as the simple substance specifically binds to the specific binding substance immobilized in the first detection zone 5 in preference to the hCG specifically bound to the colloidal gold labeled anti-hCG monoclonal antibody. This results in a decrease in the amount of the colloidal gold labeled anti-hCG bound in the first detection zone 5, thereby decreasing the signal intensity attributed to the colloidal gold. Accordingly, the signal intensity detected in the first detection zone 5 does not reflect the amount of the hCG in the sample. As described above, this phenomenon, in which the signal intensity does not increase according to the amount of the analyte, is called a prozone phenomenon.

Of the molecules of the colloidal gold labeled anti-hCG monoclonal antibody as the first specific binding substance that have reached the second detection zone 6, those capable of further binding to the hCG as the analyte specifically bind to the hCG as the immobilized analyte thereby to be immobilized in the second detection zone 6. In other words, the colloidal gold labeled anti-hCG monoclonal antibody as the first specific binding substance is immobilized in the second detection zone 6, via the hCG as the analyte.

That is, the sample having been subjected to the step (B) is brought into contact with a second detection zone where a substance identical to the analyte or an analog of the analyte is immobilized, to allow the first specific binding substance in the complex to bind to the analyte or the analog, thereby immobilizing the complex in the second detection zone (step (C)).

Herein, it is preferable that the sample develops such that the sample keeps flowing even after it migrates beyond the first detection zone 5 and the second detection zone 6. For this purpose, a sufficient amount of the sample is applied to the sample application zone 3 so that an excess of the labeled first specific binding substance, which does not participate in the specific binding reaction in the first detection zone 5 and the second detection zone 6, is allowed to be washed off from the first detection zone 5 and the second detection zone 6, by the sample flowing through the detection zones. Therefore, an absorption zone may be provided on the end of the matrix 2 where the sample develops. The material constituting the absorption zone may be any material having an absorptive property to sufficiently wash off the components other than the analyte. Examples include the glass fiber filter paper GA-200 (manufactured by TOYO KABUSHIKI KAISHA).

With the provision of the absorption zone, any unreacted substance can be washed off along with the flow of the sample, so that after the specific binding reaction, a signal attributed to the specific binding reaction can be detected in the first detection zone 5 and the second detection zone 6, without performing separation of the unreacted substance. Accordingly, in the specific binding analysis method of the present invention, it is possible to measure a signal intensity attributed to the specific binding reaction between the analyte and the specific binding substance, at any portion in a site where the specific binding reaction occurs. However, it is preferable to perform a qualitative or quantitative analysis of the analyte in the sample by measuring a signal intensity or a distribution of the signal intensity in each of the first detection zone 5 and the second detection zone 6, or in a wide region including these detection zones.

Then, an intensity of signal 1 and that of signal 2, which are attributed to the labeling material and are obtained in the first detection zone and the second detection zone, are respectively measured (step (D)), and a concentration of the analyte in the sample is determined based on the intensity of the signal 1 and that of the signal 2, thereby performing a qualitative or quantitative analysis of the analyte in the sample (step (E)).

A quantitative information showing the correlation between the distribution of the signal intensity and the concentration of an analyte, may be previously prepared using samples having known concentrations. The concentration of the analyte may also be determined based on the quantitative information and the distribution of the signal intensity obtained by the actual analysis.

FIG. 2 is a schematic diagram illustrating an analysis apparatus used along with the strip 1 in the specific binding analysis method of the present invention. The analysis device comprises, for example, a first electrode 11, a second electrode 12, a start-of-measurement detector 10, an analyzer 9, a light source 7 and a light detector 8. The first electrode 11 and the second electrode 12 are used for measuring the electrical conductivity of the sample application zone 3. The start-of-measurement detector 10 detects the application of a sample to the sample application zone 3 based on the change of the electrical conductivity, and supplies a detection signal to the analyzer 9. After an elapse of a predetermined time from the receiving of the detection signal, the analyzer 9 controls a stage 13 to scan the entire test strip 1 in the direction shown by the arrow Z. Further, the analyzer 9 controls the light source 7, and analyzes an output signal from the light detector 8. The light source 7 applies light to each of the first detection zone 5 and the second detection zone 6. The light detector 8 detects a reflected light from each of the first detection zone 5 and the second detection zone 6.

In the following, the operation of the analysis device will be described. Prior to the application of a sample, an electrical conductivity of the sample application zone 3 in the dry state and that in the wet state upon the application of a sample, are previously measured. The information thus obtained is previously inputted in the start-of-measurement detector 10 as a start-of-measurement information.

Thereafter, when a sample containing an hCG is applied to the sample application zone 3 and the sample application zone 3 subsequently turns from dry to wet, the electrical conductivity of the sample application zone 3, which is being monitored by the first electrode 11 and the second electrode 12, changes. By making reference to the change of the electrical conductivity and the start-of-measurement information, the start-of-measurement detector 10 detects the application of the sample to the sample application zone 3.

Herein, it is preferable to apply the sample to the application zone 3 after placing the test strip 1 in the specific binding analysis device, but alternatively, the test strip 1 applied with the sample may be placed in the specific binding analysis device.

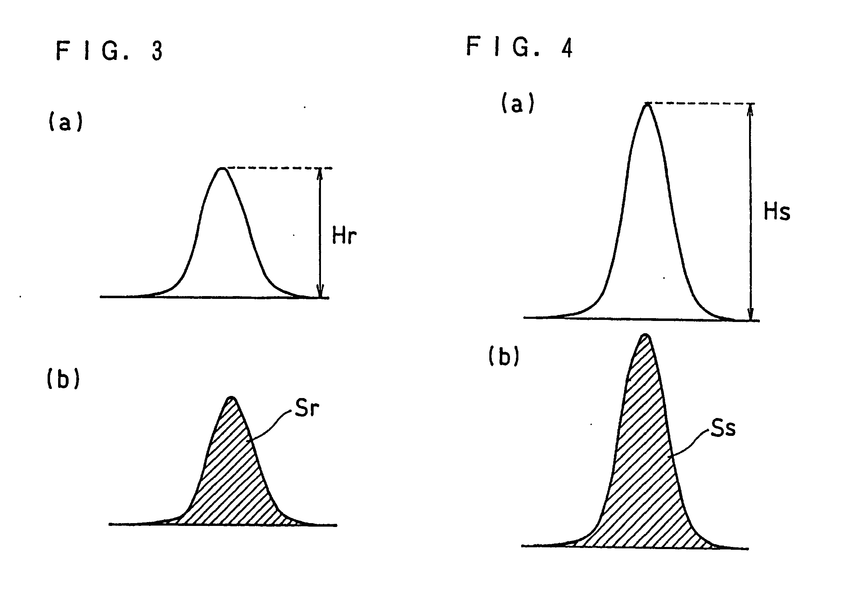

When the start-of-measurement detector 10 detects the application of the sample, the analyzer 9 controls the stage 13 to scan the test strip 1 in the direction shown by the arrow Z. At the same time, the analyzer 9 controls both the light source 7 and the light detector 8 to measure, at a predetermined time interval, the signal intensity caused by the scanning, thereby giving chromatograms shown in FIGS. 3 and 4.

The light source 7 applies light of a predetermined wavelength (e.g., 650 nm) to a region including the first detection zone 5 and the second detection zone 6, whereupon the light detector 8 detects the reflected light. As the wavelength used for the measurement, any wavelength suitable for the coloration of the sample or that of the labeling material in the first detection zone 5 and the second detection zone 6, may be appropriately selected.

The signal as used herein may be any detectable signal that can be generated by a reaction in which a labeling material participates, for example: fluorescence measurable with a fluorometer; luminescence measurable with a luminescence photometer; and coloration measurable with a visual evaluation or with a color-difference meter in the first detection zone 5 and the second detection zone 6. In this case, the intensity or the like of a reflected light, or fluorescence or luminescence generated in the first detection zone 4 and the second detection zone 6, is detected in the first detection zone 5 and the second detection zone 6.

The detection of the above-described signal is continuously performed, while relatively changing the position of the test strip 1 and the positions of the light source 7 and light detector 8 in the direction parallel to the permeating direction of the sample on the test strip 1, that is, in the direction shown by the arrow Z. Either one of the test strip 1 and the light source 7 may be moved, or both of them may be moved together, in the direction parallel to the permeating direction of the sample.

The light detector 8 sends the signal to the analyzer 9. The analyzer 9 analyzes the signal from the light detector 8, thereby measuring the intensity of the signal. Then, the analyzer 9 produces a correlation curve representing the relation between the measured signal intensity and the scanned distance, that is, a chromatogram, and analyses the same.

FIGS. 3 and 4 are schematic diagrams respectively showing the correlation curves (chromatograms) plotted by analyzing the distribution of the signal intensity in the vicinities of the first detection zone 5 and the second detection zone 6. The chromatograms were plotted by applying light from the light source 7 onto the matrix 2 while scanning the test strip 1 or the light source 7, and then analyzing a reflected light detected by the light detector 8 with the analyzer 9. In the chromatograms, the virtual vertical axis denotes the signal intensity on or in the vicinity of the first detection zone 5 or the second detection zone 6, and the horizontal axis denotes the region of the first detection zone 5 or the second detection zone 6 (the scanned distance from the sample application zone 3).

The height of each chromatogram may be measured as a signal intensity attributed to a specific binding reaction. The area of each chromatogram may also be measured as a signal intensity attributed to a specific binding reaction. Herein, the heights of the chromatograms of the first detection zone 5 and the second detection zone 6 are denoted as Hs and Hr, respectively. The areas of the chromatograms of the first detection zone 5 and the second detection zone 6 are denoted as Ss and Sr, respectively.

In this embodiment, the chromatograms are obtained by continuously measuring a reflected light in a region including the first detection zone 5 and the second detection zone 6, while scanning the test strip 1 or the light source 7. For example, a signal intensity in an upstream portion of a region including the first detection zone 5 and the second detection zone 6, i.e., a portion of the region which is closer to the sample application zone 3, is connected with a straight line to a signal intensity in a downstream portion of the region, i.e., a portion of the region which is more distant from the sample application zone 3. Then, a distance obtained by subtracting the height of the straight line from that of the highest value of the chromatogram of each of the first detection zone 5 and the second detection zone 6, may be considered as the height of the chromatogram. Also, the area of the region surrounded by the straight line and the chromatograms may be considered as the area of the chromatogram. In this manner, a signal intensity attributed to a background can be subtracted from the signal intensity in each of the first detection zone 5 and the second detection zone 6, thereby making it possible to obtain a signal intensity attributed to a specific binding reaction. Accordingly, it is possible to perform a qualitative or quantitative analysis of an analyte even in a colored sample such as whole blood, without being influenced by the background and foreign matter.

Herein, a signal intensity in an arbitrary portion on the matrix 2 other than the first detection zone 5 and the second detection zone 6 may also be considered as a signal intensity attributed to the background. For example, a signal intensity of the smallest value may be selected from signal intensities in the vicinity of each of the first detection zone 5 and the second detection zone 6.

The background as used herein refers to a behavior exhibited by a sample containing no analyte. For example, a signal intensity attributed to the nonspecific absorption of the specific binding substance or the like in each of the first detection zone 5 and the second detection zone 6, and coloration or the like of the sample itself in the case of measuring a signal attributed to the coloration, have an influence on the background, thereby reducing the measurement sensitivity. In addition, the foreign matter generally refers to a substance other than an analyte contained in a sample. The foreign matter that can cause a problem in a specific binding analysis method is a substance such as the one which exhibits a similar behavior to that of the analyte in its binding reaction with a specific binding substance (e.g., an analog of the analyte), or the one which impedes a specific binding reaction between the analyte and the specific binding substance, for example, by binding to the analyte.

The analyzer 9 contains a quantitative information in a memory (not shown), and determines the concentration of the analyte by making reference to the quantitative information as well as Hs and Hr, or Ss and Sr, each of which is the analyzed value.

In the following, the specific binding analysis method of the present invention will be described by way of an example, which was carried out using the strip shown in FIG. 1 and the analysis device shown in FIG. 2. However, the present invention is not limited thereto.

### Example

The specific binding analysis method of the present invention was carried out using the strip 1 shown in FIG. 1 and the analysis device shown in FIG. 2. Herein, the concentration of the analyte was determined from Hs and Hr by the analyzer 9.

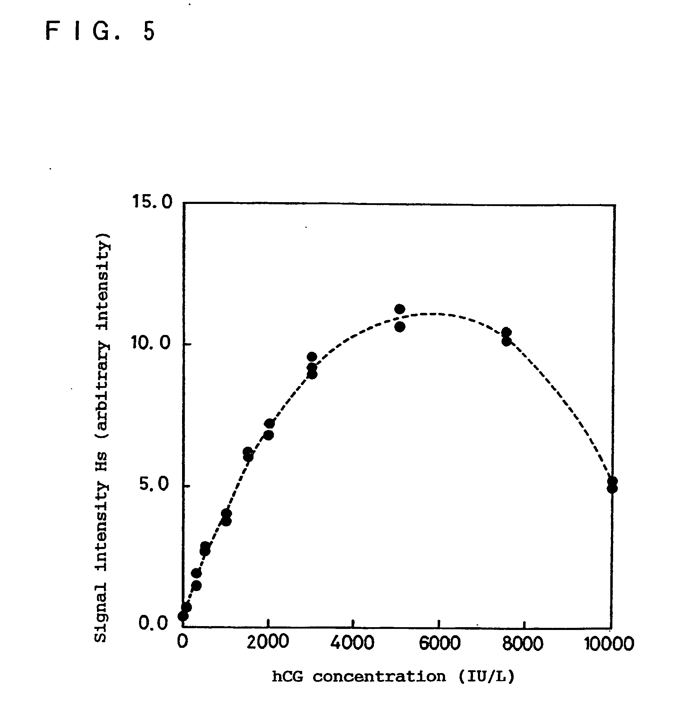

FIG. 5 shows a graph showing the relation between signal intensity Hs and the hCG concentration in the case of applying light having a wavelength of 650 nm to the first detection zone 5. The samples used herein were each prepared by adding an hCG to a control urine for accuracy control whose hCG concentration was substantially zero. The hCG concentrations of the samples were set to 0 (IU/L), 10 (IU/L), 30 (IU/L), 50 (IU/L), 100 (IU/L), 300 (IU/L), 500 (IU/L), 1000 (IU/L), 1500 (IU/L), 2000 (IU/L), 3000 (IU/L), 5000 (IU/L), 7500 (IU/L) and 10000 (IU/L), respectively. Measurement was carried out either two or three times for each sample.

As is shown from FIG. 5, in the cases of the samples having low hCG concentrations, almost all of the analytes were bound to the labeled first specific binding substance, and were bound to the second specific binding substance immobilized in the first detection zone 5. Accordingly, the signal intensity Hs, which was detected in the first detection zone 5 and was attributed to a reaction in which the labeling material participated, increased with an increase in the concentration. In the cases of the samples having high hCG concentrations, an excessive amount of the analyte not bound to the labeled first specific binding substance was present in the reaction system, so that the analyte specifically bound to the labeled first specific binding substance and the analyte not specifically bound to the labeled first specific binding substance competed to specifically bind to the second specific binding substance immobilized in the first detection zone 5. As a result, the signal intensity Hs, which was detected in the first detection zone 5 and was attributed to a reaction in which the labeling material participated, decreased with an increase in the concentration.

By using the dotted line shown in FIG. 5 as an analytical line, it was possible to determine the concentration of the analyte. More specifically, when the signal intensity Hs gave a certain value, a concentration corresponding to the certain value was determined by the horizontal axis via the dotted line. For example, when the signal intensity Hs was 5.0, the concentration could be determined as approximately 1200 (IU/L).

However, there were cases where the analyzer 9, by making reference to the dotted line in FIG. 5 as the analytical line, determined that plural concentrations were present and thus the analyte concentration corresponding to the signal intensity could not be unambiguously determined. In the case where there was a possibility that the hCG concentration could be 6000 (IU/L) or higher, it was not possible to determine whether the hCG concentration was approximately 3500 (IU/L) or approximately 7600 (IU/L) when the signal intensity Hs was 10. In such a case where the analyte concentration corresponding to the signal intensity Hs could not be unambiguously determined, it was possible to determine the concentration by obtaining the signal intensity Hr from the chromatogram of the second detection zone 6, together with the signal intensity Hs, and analyzing both the Hr and the Hs in a manner as described below.

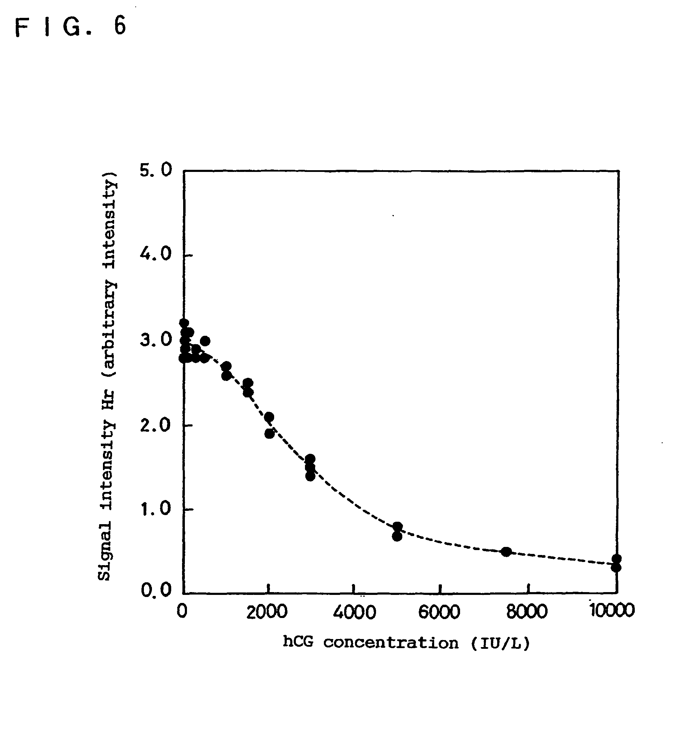

FIG. 6 shows a graph showing the relation between signal intensity Hr and the hCG concentration in the case of applying light having a wavelength of 650 nm to the second detection zone 6.

The samples used herein were each prepared by adding an hCG to a control urine for accuracy control whose hCG concentration was substantially zero. The hCG concentrations of the samples were 0 (IU/L), 10 (IU/L), 30 (IU/L), 50 (IU/L), 100 (IU/L), 300 (IU/L), 500 (IU/L), 1000 (IU/L), 1500 (IU/L), 2000 (IU/L), 3000 (IU/L), 5000 (IU/L), 7500 (IU/L) and 10000 (IU/L), respectively. Measurement was carried out two or three times for each sample.

As is shown from FIG. 6, when the concentration of the analyte in the sample was high, the labeled first specific binding substance not bound to the analyte flew from the first detection zone and bound to the analyte or analog thereof immobilized in the second detection zone 6, so that the signal intensity Hr, which was attributed to the labeling material, decreased. More specifically, when the concentration of the analyte in the sample was low and there was thus present labeled first specific binding substances not bound to the analyte, the excessive, free labeled first specific binding substances were bound to the analyte or analog thereof immobilized in the second detection zone 6. This resulted in the generation of the signal intensity Hr, which was attributed to a reaction in which the labeling material participated. On the other hand, when the concentration of the analyte in the sample was high and there was thus present an excessive amount of the analyte, substantially all of the labeled first specific substances were bound to the analyte. Even when these labeled first specific substances reached the second detection zone 6, they were unable to bind to the analyte or analog thereof immobilized in the second detection zone 6 and were washed off from the second detection zone 6. Accordingly, there was substantially no generation of the signal intensity Hr, which was attributed to a reaction in which the labeling material participated.

Herein, in the more strict sense, when the first specific binding substance had plural binding sites like an antibody (two binding sites, in the case of IgG antibody), as in this example, the binding states of the respective binding sites were reflected on the signal intensity. That is, there was a significantly low possibility that the first specific binding substance all of which binding sites were occupied by analytes would be able to further bind to an analyte. Accordingly, there was also a significantly low possibility that such first specific binding substance would bind to the analyte immobilized in the second detection zone 6. However, when there was even one possible binding site remaining for the analyte in the first specific binding substance, the possibility for this first specific binding substance to bind to the analyte immobilized in the second detection zone 6 increased with the ratio of the remaining binding site. In each case, the signal intensity Hr denoted by the dotted line in FIG. 6 decreased with an increase in the analyte concentration.

By using, as a quantitative information, the dotted lines in FIG. 6 and FIG. 5, it was possible to determine the concentration of the analyte in the following manner.

As described above, there were cases where the analyzer 9, by making reference to the dotted line of FIG. 5, determined that plural concentrations were present and thus the analyte concentration corresponding to the signal intensity could not be unambiguously determined. In the case where there was a possibility that the hCG concentration could be 6000 (IU/L) or higher, it was not possible to determine whether the hCG concentration was approximately 3500 (IU/L) or approximately 7600 (IU/L) when the signal intensity Hs was 10. However, it was possible to determine the concentration by further making reference to the dotted line in FIG. 6. More specifically, when the signal intensity Hr was 1.2 in FIG. 6, the concentration could be determined as approximately 3500 (IU/L) from the dotted line. When the signal intensity Hr was 0.45 in FIG. 6, the concentration could be determined as approximately 7600 (IU/L) from the dotted line.

As such, even in the case where the analyte concentration could not be unambiguously determined only from the signal intensity Hs in FIG. 5, it was possible to determine the concentration by obtaining the signal intensity Hr from the chromatogram of the second detection zone 6 and analyzing both the Hr and the Hs.

Herein, it was possible to determine the concentration only from the Hr in FIG. 6. However, the measurement accuracy was lower when the concentration was determined from the Hr than when it was determined from the Hs, since the gradient of the dotted line with respect to the concentration was small in the low concentration range. Accordingly, it was preferable to employ the Hr only for determining either one of two concentrations derived from the Hs was true, while employing the Hs alone for determining the concentration.

Additionally, it was possible to simplify the measurement by determining the concentration from the Hs in FIG. 5 only when the Hr in FIG. 6 was greater than a predetermined value. In the case of this example, the measurement was simplified by, for instance, determining the concentration from the Hs in FIG. 5 only when the Hr was 0.6 or greater, and judging that the prozone phenomenon was present when the Hr was 0.6 or less and merely limiting the concentration to 6000 (IU/L) or higher, without determining a specific amount of the concentration. That is, employing the Hr only for judging whether or not the prozone phenomenon was present, was effective in simplifying the measurement.

In this example, the second detection zone 6 was placed downstream from the first detection zone 5 in the developing direction of the liquid sample (the direction shown by the arrow Z). Such placement improved the accuracy of the quantitative analysis for the following reason. When a liquid sample flowed through a matrix wherein protein such as an antibody was immobilized, the flow of the liquid sample was impeded to induce a phenomenon in which the liquid sample flowed only through a particular portion of the matrix, thereby resulting in a inhomogeneous binding in the detection zone in the downstream side. Consequently, the measurement reproducibility was reduced. The impeding of the liquid sample flow by the protein immobilized in the matrix was induced by a spatial barrier, nonuniform hydrophilicity caused by inhomogeneous immobilization density and the like. Since the measurement accuracy was improved by ensuring the reproducibility of the signal for determining, that is, quantitatively analyzing the concentration, which was generated in the detection zone 5, it was preferable to place the detection zone for quantitative analysis in the upstream side.

As described above, analyzing both the Hs and the Hr as a quantitative information made it possible to determine the concentration, without being influenced by the so-called prozone phenomenon.

It should be noted that while this example employed the Hs and the Hr, employing the Ss and the Sr shown in FIGS. 3 and 4 achieved similar results. More specifically, the Ss was employed for the quantitative determination, while the Sr was employed for the determination whether or not the prozone phenomenon was present.

As described above, according to the specific binding analysis method of this example, it was possible to perform a qualitative or quantitative analysis of the analyte contained in the sample in a simple and rapid manner, even when the prozone phenomenon was present.

As set forth above, according to the present invention, the amount of an analyte in a sample can be determined by making reference to a quantitative information showing the correlation between the concentration of the analyte and the analyzed values of signal intensities which are attributed to the specific binding reaction of specific binding substances and are generated in two detection zones, thereby enabling an accurate qualitative or quantitative analysis to be performed in a simple and rapid manner, without being influenced by the prozone phenomenon.

Although the present invention has been described in terms of the presently preferred embodiments, it is to be understood that such disclosure is not to be interpreted as limiting. Various alterations and modifications will no doubt become apparent to those skilled in the art to which the present invention pertains, after having read the above disclosure. Accordingly, it is intended that the appended claims be interpreted as covering all alterations and modifications as fall within the scope of the invention.

To enable accurate qualitative and quantitative analyses of an analyte in a sample without being influenced by a prozone phenomenon, there is disclosed a specific binding method including the steps of: bringing into contact a sample with a zone where a first specific binding substance labeled with a labeling material is retained, to allow an analyte to bind to the first specific binding substance; bringing into contact the sample with a first detection zone where a second specific binding substance is immobilized, to allow an excess of the analyte to bind to the second specific binding substance; bringing into contact the sample with a second detection zone where a substance identical to the analyte is immobilized, to allow the first specific binding substance to bind to the substance; respectively measuring the intensities of signal 1 and signal 2 obtained in the first detection zone and the second detection zone and attributed to the labeling material; and then determining the concentration of the analyte based on the intensities.

## Claims

1. A specific binding analysis method comprising the steps of:
(A) bringing into contact a sample containing an analyte with a first specific binding substance which specifically binds to the analyte and is labeled with a detectable labeling material, to allow said analyte to bind to said first specific binding substance, thereby obtaining a labeling material-first specific binding substance-analyte complex;
(B) bringing into contact said sample subjected to step (A) with a first detection zone (5) where a second specific binding substance is immobilized, to allow an excess of said analyte, which has not bound to said first specific binding substance, and said complex to bind to said second specific binding substance, thereby immobilizing said excess of said analyte and said complex in said first detection zone (5);
(C) bringing into contact said sample subjected to step (B) with a second detection zone (6) where a substance identical to said analyte or an analog of said analyte is immobilized, to allow said first specific binding substance in said complex to bind to said substance or said analog, thereby immobilizing said complex in said second detection zone (6);
(D) respectively measuring an intensity of signal 1 and an intensity of signal 2, which are attributed to said labeling material and are obtained in said first detection zone (5) and said second detection zone (6); and
(E) determining a concentration of said analyte in said sample based on said intensity of said signal 1 and said intensity of said signal 2, thereby performing a qualitative or quantitative analysis of said analyte in said sample.

2. The specific binding analysis method in accordance with claim 1,
wherein, in said step (E), when said intensity of said signal 1 obtained in said step (D) corresponds to different concentrations of said analyte based on quantitative information previously obtained from a sample having a known concentration of the analyte, a concentration of said analyte is determined after judging which concentration is true based on said intensity of said signal 2.

3. The specific binding analysis method in accordance with claim 1 or 2, further comprising
judging whether or not a prozone phenomenon is present based on said intensity of said signal 2 obtained in said step (D).

4. The specific binding analysis method in accordance with claim 1,
wherein, in said step (B), said sample having been subjected to said step (A) is brought into contact with a first detection zone (5) where a second specific binding substance is immobilized, to allow an excess of said analyte, which has not bound to said first specific binding substance, and said complex to bind to said second specific binding substance, thereby immobilizing said excess of said analyte and said complex in said first detection zone (5), and
in said step (C), said sample having been subjected to said step (B) is brought into contact with a second detection zone (6) where a substance identical to said analyte or an analog of said analyte is immobilized, to allow said first specific binding substance in said complex to bind to said substance or said analog, thereby immobilizing said complex in said second detection zone (6), said step (C) being performed subsequently to said step (B).

5. The specific binding analysis method in accordance with claim 1,
wherein, in said step (C), said sample having been subjected to said step (A) is brought into contact with a second detection zone (6) where a substance identical to said analyte or an analog of said analyte is immobilized, to allow said first specific binding substance in said complex to bind to said substance or said analog, thereby immobilizing said complex in said second detection zone (6), and
in said step (B), said sample having been subjected to said step (C) is brought into contact with a first detection zone (5) where a second specific binding substance is immobilized, to allow an excess of said analyte, which has not bound to said first specific binding substance, and
said complex to bind to said second specific binding substance, thereby immobilizing said excess of said analyte and said complex in said first detection zone (5),
said step (B) being performed subsequently to said step (C).

6. The specific binding analysis method in accordance with claim 1,
wherein, after previously performing said step (A) and providing said first detection zone (5) and said second detection zone (6) on a sheet-shaped chromatography matrix (2), said sample having been subjected to said step (A) is dropped upstream from said first detection zone (5) and said second detection zone (6), to allow said sample to migrate, by permeating force caused by capillarity, to each of said first detection zone (5) and said second detection zone (6) on said matrix (2), followed by performing said steps (B) to (E).

7. The specific binding analysis method in accordance with claim 1,
wherein, after providing a retention zone (4) where a first specific binding substance labeled with a detectable labeling material is retained, said first detection zone (5) and said second detection zone (6) on a sheet-shaped chromatography matrix (2), said sample is dropped to said retention zone (4) or upstream therefrom, to allow said sample to migrate, by permeating force caused by capillarity, to each of said retention zone (4), said first detection zone (5) and said second detection zone (6) on said matrix (2), followed by performing said steps (A) to (E).

8. The specific binding analysis method in accordance with claim 1,
wherein said signal attributed to said labeling material is a signal exhibiting coloration, fluorescence or luminescence.

9. The specific binding analysis method in accordance with claim 1,
wherein at least one of said first specific binding substance and said second specific binding substance is an antibody.

10. The specific binding analysis method in accordance with claim 1,
wherein said labeling material is a particle containing a metal sol, a dye sol or a fluorescent substance, or a colored latex particle.

11. The specific binding analysis method in accordance with claim 1,
wherein, in said step (D), said intensity of said signal 1 and said intensity of said signal 2 are continuously measured in this order, along the migrating direction of said analyte.

12. The specific binding analysis method in accordance with claim 11,
wherein, in said step (D), a graph showing the relation between a position of said analyte in the migrating direction thereof and each of said intensity of said signal 1 and said intensity of said signal 2 each measured continuously, is produced, and
in said step (E), a qualitative or quantitative analysis is performed based on said graph.

13. The specific binding analysis method in accordance with claim 1,
wherein, in said step (E), a quantitative analysis is performed based on a quantitative information previously obtained from a sample having a known concentration of an analyte, as well as on said intensity of said signal 1 and said intensity of said signal 2 each obtained in said step (D).

14. A specific binding analysis device comprising:
a sheet-shaped chromatography matrix (2);
a first detection zone (5) where a specific binding substance which specifically binds to an analyte is immobilized;
a second detection zone (6) where a substance identical to said analyte or an analog of said analyte is immobilized, each of said first detection zone (5) and said second detection zone (6) being provided on said matrix (2),
wherein said device being adapted such that a sample containing an analyte and a specific binding substance migrates by permeating force caused by capillarity, to each of said first detection zone (5) and said second detection zone (6) to cause a specific binding reaction, and a signal attributed to said specific binding reaction is detected to perform a qualitative or quantitative analysis of said analyte in said sample.

15. The specific binding analysis device in accordance with claim 14,
further comprising a retention zone (4) where a specific binding substance labeled with a detectable labeling material is retained, said retention zone (4) being provided upstream from said first detection zone (5) on said matrix (2),
wherein said device being adapted such that said sample migrates by permeating force caused by capillarity, from said retention zone (4) to each of said first detection zone (5) and said second detection zone (6).

16. The specific binding analysis device in accordance with claim 15,
further comprising a sample application zone (3) provided upstream from said retention zone (4) on said matrix (2),
wherein said device being adapted such that said sample migrates by permeating force caused by capillarity, from said sample application zone (3) to each of said retention zone (4), said first detection zone (5) and said second detection zone (6).

17. The specific binding analysis device in accordance with claim 14,
further comprising a sample application zone (3) provided upstream from said first detection zone (5) on said matrix (2),
wherein said device being adapted such that said sample migrates, by permeating force caused by capillarity, from said sample application zone (3) to each of said first detection zone (5) and said second detection zone (6).

## Patentansprüche

1. Spezifisches Bindungsanalyseverfahren mit den Schritten von:
(A) In Kontakt bringen einer Probe, die ein Analyt enthält, mit einer ersten spezifischen Bindungssubstanz, welche spezifisch an das Analyt bindet und mit einem nachweisbaren Markierungsmaterial markiert ist, um es dem Analyt zu ermöglichen, an die erste spezifische Bindungssubstanz zu binden, wodurch ein Komplex aus dem Markierungsmaterial, der ersten spezifischen Bindungssubstanz und dem Analyt erhalten wird;
(B) In Kontakt bringen der dem Schritt (A) unterzogenen Probe mit einer ersten Nachweiszone (5), in der eine zweite spezifische Bindungssubstanz immobilisiert ist, um es einem Überschuss des Analyts, der nicht an die erste spezifische Bindungssubstanz gebunden wurde, zu ermöglichen, an die zweite spezifische Bindungssubstanz zu binden, wodurch der Überschuss des Analyts und der Komplex in der ersten Nachweiszone (5) immobilisiert werden;
(C) In Kontakt bringen der dem Schritt (B) unterzogenen Probe mit einer zweiten Nachweiszone (6), in der eine mit dem Analyt identische Substanz oder ein Analogon des Analyts immobilisiert ist, um es der ersten spezifischen Bindungssubstanz in dem Komplex zu ermöglichen, an die Substanz oder das Analogon zu binden, wodurch der Komplex in der zweiten Nachweiszone (6) immobilisiert wird;
(D) Entsprechende Messung einer Intensität des Signals 1 und einer Intensität des Signals 2, welche auf das Markierungsmaterial zurückzuführen sind und in der ersten Nachweiszone (5) und der zweiten Nachweiszone (6) erhalten werden; und
(E) Bestimmen einer Konzentration des Analyts in der Probe auf der Grundlage der Intensität des Signals 1 und der Intensität des Signals 2, wodurch eine qualitative oder quantitative Analyse des Analyts in der Probe durchgeführt wird.

2. Spezifisches Bindungsanalyseverfahren nach Anspruch 1,
wobei in Schritt (E), wenn die Intensität des in Schritt (D) erhaltenen Signals 1 unterschiedlichen Konzentrationen des Analyts auf der Grundlage von einer früher erhaltenen Information einer Probe, die eine bekannte Konzentration hat, entspricht, eine Konzentration des Analyts bestimmt wird, nachdem auf der Grundlage der Intensität des Signals 2 entschieden wurde, welche Konzentration richtig ist.

3. Spezifisches Bindungsanalyseverfahren nach Anspruch 1 oder 2, das zusätzlich die Entscheidung umfasst, ob auf der Grundlage der Intensität des in Schritt (D) erhaltenen Signals 2 ein Prozonenphänomen vorhanden ist oder nicht.

4. Spezifisches Bindungsanalyseverfahren nach Anspruch 1,
wobei in Schritt (B) die dem Schritt (A) unterzogene Probe mit einer ersten Nachweiszone (5) in Kontakt gebracht wird, in der eine zweite spezifische Bindungssubstanz immobilisiert ist, um es einem Überschuss des Analyts, welcher nicht an die erste spezifische Bindungssubstanz gebunden wurde, um dem Komplex zu ermöglichen, an die zweite spezifische Bindungssubstanz zu binden, wodurch der Überschuss des Analyts und der Komplex in der ersten Nachweiszone immobilisiert werden,
und
in dem Schritt (C) die Probe, die dem Schritt (B) unterzogen wurde, in Kontakt mit einer zweiten Nachweiszone (6) gebracht wird, in der eine mit dem Analyt oder dem Analogon des Analyts identische Substanz immobilisiert ist, um es der ersten spezifischen Bindungssubstanz in dem Komplex ermöglicht, an die Substanz oder das Analogon zu binden, wodurch der Komplex in der zweiten Nachweiszone (6) immobilisiert wird, wobei der Schritt (C) nach dem Schritt (B) durchgeführt wird.

5. Spezifisches Bindungsanalyseverfahren nach Anspruch 1,
wobei in dem Schritt (C) die Probe, die dem Schritt
(A) unterzogen wurde, in Kontakt mit einer zweiten Nachweiszone (6) gebracht wird, in der eine Substanz, die mit dem Analyt oder dem Analogon des Analyts identisch ist, immobilisiert ist, um es der ersten spezifischen Bindungssubstanz in dem Komplex zu ermöglichen, an die Substanz oder das Analogon zu binden, wodurch der Komplex in der zweiten Nachweiszone (6) immobilisiert wird, und
in dem Schritt (B) die Probe, die dem Schritt (C) unterzogen wurde, in Kontakt mit der ersten Nachweiszone (5) gebracht wird, in der eine zweite spezifische Bindungssubstanz immobilisiert ist, um es dem Überschuss des Analyts, welcher nicht an die erste spezifische Bindungssubstanz gebunden wurde, und dem Komplex zu ermöglichen, an die zweite spezifische Bindungssubstanz zu binden, wodurch der Überschuss des Analyts und der Komplex in der ersten spezifischen Bindungszone (5) immobilisiert werden,
wobei der Schritt (B) nach dem Schritt (C) durchgeführt wird.

6. Spezifisches Bindungsanalyseverfahren nach Anspruch 1,
wobei, nachdem vorher der Schritt (A) durchgeführt und die erste (5) und die zweite Nachweiszone (6) auf einer blattförmigen Chromatographiematrix (2) vorgesehen werden, die Probe, die Schritt (A) unterzogen wurde, stromaufwärts der ersten Nachweiszone (5) und der zweiten Nachweiszone (6) aufgetropft wird, um es der Probe durch die durch Kapillarität verursachte Durchdringungskraft zu ermöglichen, zu jeder der ersten Nachweiszone (5) und der zweiten Nachweiszone (6) auf der Matrix (2) zu migrieren, gefolgt von der Durchführung der Schritte (B) bis (E).

7. Spezifisches Bindungsanalyseverfahren nach Anspruch 1,
wobei nach Vorsehen einer Retentionszone (4), in der eine erste spezifische Bindungssubstanz, die mit einem nachweisbaren Markierungsmaterial markiert ist, aufbewahrt wird, der ersten Nachweiszone (5) und der zweiten Nachweiszone (6) auf einer blattförmigen Chromatographiematrix (2), die Probe auf die Retentionszone oder stromaufwärts davon aufgetropft wird, um es der Probe durch die durch Kapillarität verursachte Durchdringungskraft zu ermöglichen, zu jeder der Retentionszone 4, der ersten Nachweiszone (5) und der zweiten Nachweiszone (6) auf der Matrix (2) zu migrieren, gefolgt von der Durchführung der Schritte (A) bis (E).

8. Spezifisches Bindungsanalyseverfahren nach Anspruch 1,
wobei das Signal, das dem Markierungsmaterial eigen ist, ein Signal ist, das eine Färbung, eine Fluoreszenz oder eine Lumineszenz aufweist.

9. Spezifisches Bindungsanalyseverfahren nach Anspruch 1,
wobei wenigstens eine der ersten und der zweiten spezifischen Bindungssubstanzen ein Antikörper ist.

10. Spezifisches Bindungsanalyseverfahren nach Anspruch 1,
wobei das Markierungsmaterial ein Teilchen ist, das ein Metall-Sol, ein Farbstoff-Sol oder eine fluoreszierende Substanz oder ein gefärbtes Latexteilchen enthält.

11. Spezifisches Bindungsanalyseverfahren nach Anspruch 1,
wobei in Schritt (D) die Intensität des Signals 1 und die Intensität des Signals 2 kontinuierlich in der Reihenfolge entlang der Migrationsrichtung des Analyts gemessen werden.

12. Spezifisches Bindungsanalyseverfahren nach Anspruch 11,
wobei in Schritt (D) eine grafische Darstellung erzeugt wird, die die Beziehung zwischen einer Position des Analyts in dessen Migrationsrichtung und jeder der Intensitäten des Signals 1 und des Signals 2, die jeweils kontinuierlich gemessen werden, zeigt, und wobei in dem Schritt (E) eine qualitative oder quantitative Analyse auf der Grundlage der grafischen Darstellung durchgeführt wird.

13. Spezifisches Bindungsanalyseverfahren nach Anspruch 1,
wobei in dem Schritt (E) eine quantitative Analyse auf der Grundlage einer quantitativen Information, die vorher von einer Probe, die eine bekannte Konzentration eines Analyts hat, erhalten wurde, sowohl für die Intensität des Signals 1 als auch die Intensität des Signals 2, die jeweils in Schritt (D) erhalten wurden, durchgeführt wird.

14. Spezifische Bindungsanalysevorrichtung, die umfasst:
eine blattförmige Chromatographiematrix (2);
eine erste Nachweiszone (5), in der eine spezifische Bindungssubstanz, welche spezifisch an ein Analyt bindet, immobilisiert ist;
eine zweite Nachweiszone (6), in der eine Substanz, die dem Analyt identisch ist, oder ein Analogon des Analyts, immobilisiert ist, wobei jeder der ersten Nachweiszone (5) und der zweiten Nachweiszone (6) auf der Matrix (2) zur Verfügung gestellt werden,
wobei die Vorrichtung so angepasst ist, dass eine Probe, die ein Analyt und eine spezifische Bindungssubstanz enthält, durch die durch Kapillarität verursachte Durchdringungskraft zu jeder der ersten Nachweiszone (5) und der zweiten Nachweiszone (6) migriert, um eine spezifische Bindungsreaktion zu verursachen, und ein Signal, das auf die spezifische Bindungsreaktion zurückzuführen ist, nachgewiesen wird, um eine qualitative oder quantitative Analyse des Analyts in der Probe durchzuführen.

15. Spezifische Bindungsanalysevorrichtung nach Anspruch 14,
welche zusätzlich eine Retentionszone (4) aufweist, in der eine spezifische Bindungssubstanz, die mit einem nachweisbaren Markierungsmaterial markiert wurde, zurückgehalten wird, wobei die Retentionszone (4) stromaufwärts der ersten Nachweiszone (5) auf der Matrix (2) vorgesehen ist,
wobei die Vorrichtung so angepasst ist, dass die Probe durch die durch Kapillarität verursachte Durchdringungskraft von der Retentionszone (4) zu jeder der ersten Nachweiszone (5) und der zweiten Nachweiszone (6) migriert.

16. Spezifische Bindungsanalysevorrichtung nach Anspruch 15,
welche zusätzlich eine Probenapplikationszone (3) enthält, die stromaufwärts der Retentionszone (4) auf der Matrix (2) vorgesehen ist, wobei die Vorrichtung so angepasst ist, dass die Probe durch die durch Kapillarität verursachte Durchdringungskraft von der Probenapplikationszone (3) zu jeder der Retentionszone (4), der ersten Nachweiszone (5) und der zweiten Nachweiszone (6) migriert.

17. Spezifische Bindungsanalysevorrichtung nach Anspruch 14,
welche zusätzlich eine Probenapplikationszone (3) enthält, die stromaufwärts der ersten Nachweiszone (5) auf der Matrix (2) angebracht ist,
wobei die Vorrichtung so angepasst ist, das die Probe, durch die durch Kapillarität verursachte Durchdringungskraft von der Probenapplikationszone (3) zu jeder der ersten (5) und der zweiten Nachweiszone (6) migriert.

## Revendications

1. Procédé d'analyse de liaisons spécifiques contenant les étapes de :
(A) mise en contact d'un échantillon contenant un analyte avec une première substance de liaison spécifique qui se lie de manière spécifique à l'analyte et est marquée avec un matériau de liaison détectable, pour permettre au dit analyte de se lier à ladite première substance de liaison spécifique, obtenant de là un complexe matériau de marquage-première substance de liaison spécifique-analyte ;
(B) mise en contact dudit échantillon soumis à l'étape (A) avec une première zone de détection (5) où une seconde substance de liaison spécifique est immobilisée, pour permettre un excès dudit analyte, qui ne s'est pas lié à ladite première substance de liaison spécifique, et au dit complexe pour se lier à ladite seconde substance de liaison spécifique, immobilisant de là ledit excès dudit analyte et ledit complexe dans ladite première zone de détection (5) ;
(C) mise en contact dudit échantillon soumis à l'étape (B) avec une seconde zone de détection (6) où une substance identique au dit analyte ou un analogue dudit analyte est immobilisée, pour permettre à ladite première substance de liaison spécifique dans ledit complexe de se lier à ladite substance ou au dit analogue, immobilisant de là ledit complexe dans ladite seconde zone de détection (6) ;
(D) mesure de manière respective d'une intensité de signal 1 et d'une intensité de signal 2, qui sont attribués au dit matériau de marquage et qui sont obtenus dans ladite première zone de détection (5) et ladite seconde zone de détection (6) ; et
(E) détermination d'une concentration dudit analyte dans ledit échantillon basée sur ladite intensité dudit signal 1 et sur ladite intensité dudit signal 2, effectuant de là une analyse qualitative ou quantitative dudit analyte dans ledit échantillon.

2. Procédé d'analyse de liaisons spécifiques conformément à la revendication 1,
dans lequel, dans ladite étape (E), lorsque ladite intensité dudit signal 1 obtenu dans ladite étape (D) correspond à différentes concentrations dudit analyte basées sur l'information quantitative précédemment obtenue à partir d'un échantillon ayant une concentration connue d'analyte, une concentration dudit analyte est déterminée après appréciation de la concentration qui est vraie sur la base de ladite intensité dudit signal 2.

3. Procédé d'analyse de liaisons spécifiques conformément à la revendication 1 ou 2, comprenant en outre
l'appréciation du fait qu'un phénomène de prozone est présent ou non sur la base de ladite intensité dudit signal 2 obtenu dans ladite étape (D).

4. Procédé d'analyse de liaisons spécifiques conformément à la revendication 1,
dans lequel, dans ladite étape (B), ledit échantillon ayant été soumis à ladite étape (A) est mis en contact avec une première zone de détection (5) où une seconde substance de liaison spécifique est immobilisée, pour permettre à un excès dudit analyte, qui n'était pas lié à ladite première substance de liaison spécifique, et au dit complexe de se lier à ladite seconde substance de liaison spécifique, immobilisant de là ledit excès dudit analyte et ledit complexe dans ladite première zone de détection (5), et dans ladite étape (C), ledit échantillon ayant été soumis à ladite étape (B) est mis en contact avec une seconde zone de détection (6) où une substance identique au dit analyte ou au dit analogue dudit analyte est immobilisée, pour permettre à ladite première substance de liaison spécifique dans ledit complexe de se lier à ladite substance ou ledit analogue, immobilisant de là ledit complexe dans ladite seconde zone de détection (6), ladite étape (C) étant effectuée ultérieurement à ladite étape (B).

5. Procédé d'analyse de liaisons spécifiques conformément à la revendication 1,
dans lequel, dans ladite étape (C), ledit échantillon ayant été soumis à ladite étape (A) est mis en contact avec une seconde zone de détection (6) où une substance identique au dit analyte ou d'un analogue dudit analyte est immobilisée, pour permettre à ladite première substance de liaison spécifique dans ledit complexe de se lier à ladite substance ou au dit analogue, immobilisant de là ledit complexe dans ladite seconde zone de détection (6), et
dans ladite étape (B), ledit échantillon ayant été soumis à ladite étape (C) est mis en contact avec une première zone de détection (5) où une seconde substance de liaison spécifique est immobilisée, pour permettre à un excès dudit analyte, qui n'était pas lié à ladite première substance de liaison spécifique, et au dit complexe de se lier à ladite seconde substance de liaison spécifique, immobilisant de là ledit excès dudit analyte et ledit complexe dans ladite première zone de détection (5), ladite étape (B) étant effectuée ultérieurement à ladite étape (C).

6. Procédé d'analyse de liaisons spécifiques conformément à la revendication 1,
dans lequel, après avoir effectué antérieurement ladite étape (A) et fourni ladite première zone de détection (5) et ladite seconde zone de détection (6) sur une matrice pour chromatographie en forme de feuille (2), ledit échantillon ayant été soumis à ladite étape (A) est laissé tomber de ladite première zone de détection (5) et ladite seconde zone de détection (6), pour permettre au dit échantillon de migrer, par la force de perméation provoquée par la capillarité, vers chacune de ladite première zone de détection (5) et de ladite seconde zone de détection (6) sur ladite matrice (2), suivi par l'exécution desdites étapes (B) à (E).

7. Procédé d'analyse de liaisons spécifiques conformément à la revendication 1,
dans lequel, après avoir fourni une zone de rétention (4) où une première substance de liaison spécifique marquée à l'aide d'un matériau de marquage détectable est retenue, ladite première zone de détection (5) et ladite seconde zone de détection (6) sur une matrice pour chromatographie en forme de feuille (2), ledit échantillon est laissé tomber vers ladite zone de rétention (4) ou en amont de celle-ci, pour permettre au dit échantillon de migrer, par la force de perméation provoquée par la capillarité, vers chacune de ladite zone de rétention (4), ladite première zone de détection (5) et ladite seconde zone de détection (6) sur ladite matrice (2), suivi par l'exécution desdites étapes (A) à (E).

8. Procédé d'analyse de liaisons spécifiques conformément à la revendication 1,
dans lequel ledit signal attribué au dit matériau de marquage est un signal faisant preuve de coloration, de fluorescence ou de luminescence.

9. Procédé d'analyse de liaisons spécifiques conformément à la revendication 1,
dans lequel au moins l'une de ladite première substance de liaison spécifique et ladite seconde substance de liaison spécifique est un anticorps.

10. Procédé d'analyse de liaisons spécifiques conformément à la revendication 1,
dans lequel ledit matériau de marquage est une particule contenant un sol de métal, un sol colorant ou une substance fluorescente, ou une particule de latex colorée.

11. Procédé d'analyse de liaisons spécifiques conformément à la revendication 1,
dans lequel, dans ladite étape (D), ladite intensité dudit signal 1 et ladite intensité dudit signal 2 sont continuellement mesurées dans cet ordre, dans le sens de migration dudit analyte.

12. Procédé d'analyse de liaisons spécifiques conformément à la revendication 11,
dans lequel, dans ladite étape (D), un graphe montrant la relation entre une position dudit analyte dans le sens de migration de celui-ci et chacune de ladite intensité dudit signal 1 et de ladite intensité dudit signal 2 chacune mesurée continuellement, est produit, et
dans ladite étape (E), une analyse qualitative ou quantitative est effectuée sur la base dudit graphe.

13. Procédé d'analyse de liaisons spécifiques conformément à la revendication 1,
dans lequel, dans ladite étape (E), une analyse quantitative est effectuée sur la base d'une information quantitative précédemment obtenue à partir d'un échantillon ayant une concentration connue d'un analyte, ainsi que sur ladite intensité dudit signal 1 et ladite intensité dudit signal 2 obtenue chacune dans ladite étape (D).

14. Dispositif d'analyse de liaisons spécifiques comprenant :
une matrice pour chromatographie en forme de feuille (2) ;
une première zone de détection (5) où une substance de liaison spécifique qui se lie spécifiquement à un analyte est immobilisée ;
une seconde zone de détection (6) où une substance identique au dit analyte ou un analogue dudit analyte est immobilisé(e), chacune de ladite première zone de détection (5) et de ladite seconde zone de détection (6) étant fournie sur ladite matrice (2),
dans lequel ledit dispositif étant adapté de sorte qu'un échantillon contenant un analyte et une substance de liaison spécifique migrent par la force de perméation provoquée par la capillarité, vers chacune de ladite première zone de détection (5) et de ladite seconde zone de détection (6) pour provoquer une réaction de liaison spécifique, et un signal attribué à ladite réaction de liaison spécifique est détecté pour effectuer une analyse qualitative ou quantitative dudit analyte dans ledit échantillon.

15. Dispositif d'analyse de liaisons spécifiques conformément à la revendication 14,
comprenant en outre une zone de rétention (4) où une substance de liaison spécifique marquée à l'aide d'un matériau de marquage détectable est retenue, ladite zone de rétention (4) étant fournie en amont de ladite première zone de détection (5) sur ladite matrice (2),
dans lequel ledit dispositif étant adapté de sorte que ledit échantillon migre par la force de perméation provoquée par la capillarité, de ladite zone de rétention (4) vers chacune de ladite première zone de détection (5) et ladite seconde zone de détection (6).

16. Dispositif d'analyse de liaisons spécifiques conformément à la revendication 15,
comprenant en outre une zone d'application d'échantillon (3) fournie en amont de ladite zone de rétention (4) sur ladite matrice (2),
dans lequel ledit dispositif étant adapté de sorte que ledit échantillon migre par la force de perméation provoquée par la capillarité, de ladite zone d'application d'échantillon (3) vers chacune de ladite zone de rétention (4), ladite première zone de détection (5) et ladite seconde zone de détection (6).

17. Dispositif d'analyse de liaisons spécifiques conformément à la revendication 14,
comprenant en outre une zone d'application d'échantillon (3) fournie en amont de ladite première zone de détection (5) sur ladite matrice (2),
dans lequel ledit dispositif étant adapté de sorte que ledit échantillon migre, par la force de perméation provoquée par la capillarité, de ladite zone d'application d'échantillon (3) vers chacune de ladite première zone de détection (5) et ladite seconde zone de détection (6).
